(19) Europäisches Patentamt
European Patent Office
Office européen des brevets

(11) EP 1 726 293 B1

(12) FASCICULE DE BREVET EUROPEEN

(45) Date de publication et mention de la délivrance du brevet:
**13.01.2010 Bulletin 2010/02**

(21) Numéro de dépôt: **06113856.6**

(22) Date de dépôt: **12.05.2006**

(51) Int Cl.:
***A61K 8/36*** *(2006.01)* ***A61K 8/44*** *(2006.01)*
***A61K 8/45*** *(2006.01)* ***A61K 8/46*** *(2006.01)*
***A61K 8/898*** *(2006.01)* ***A61K 8/73*** *(2006.01)*
***A61Q 5/02*** *(2006.01)* ***A61Q 5/12*** *(2006.01)*
***A61Q 1/14*** *(2006.01)*

# (54) Compositions cosmétiques détergentes comprenant une silicone aminée et utilisation

Kosmetische Waschmittelzusammensetzungen enthaltend ein Aminosilikon und ihre Verwendung

Cosmetic detergent compositions comprising an aminosilicon and use thereof

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **24.05.2005 FR 0551349**

(43) Date de publication de la demande:
**29.11.2006 Bulletin 2006/48**

(73) Titulaire: **L'ORÉAL**
**75008 Paris (FR)**

(72) Inventeurs:
- **Decoster, Sandrine**
**95210 Saint Gratien (FR)**
- **Meralli, Sabina**
**92170 Vanves (FR)**

(74) Mandataire: **Le Blainvaux Bellegarde, Françoise**
**L'Oréal**
**D.I.P.I.**
**25-29 Quai Aulagnier**
**92600 Asnières (FR)**

(56) Documents cités:
**EP-A- 0 739 625**
**EP-A- 0 974 335**
**WO-A-97/46211**
**US-A- 6 153 569**
**US-A1- 2003 134 760**
**US-A1- 2003 147 842**
**US-A1- 2003 157 049**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 726 293 B1

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, au moins un tensioactif anionique sulfate ou sulfonate, au moins un agent tensioactif anionique carboxylique différent du précédent, au moins un tensioactif amphotère et au moins une silicone aminée. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenus dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétée, les fibres capillaires. Ces agents conditionneurs peuvent bien entendu également améliorer le comportement cosmétique des cheveux naturels.

Pour tenter de résoudre ce problème, l'homme de l'art utilise déjà, dans des compositions de soin des matières kératiniques telles que des cheveux, des agents conditionneurs insolubles tels que les silicones.

Cependant, si ces compositions permettent, dans une certaine mesure, d'améliorer le démêlage et la douceur des cheveux, elles s'accompagnent malheureusement également, sur cheveux, de certains effets cosmétiques jugés indésirables, à savoir un alourdissement de la coiffure (manque de légèreté des cheveux), un manque de lissage (cheveux non homogènes de la racine à la pointe) et une insuffisance concernant la brillance.

De plus, ces agents conditionneurs insolubles ne permettent généralement pas d'obtenir des compositions transparentes ou limpides.

US2003/134760 décrit des compositions detergents comprenant un tensioactif anionique, un tensioactif anionique carboxylique dans un rapport inférieur à 2 et une silicone aminée. Les documents EP 0739 625, WO97/4622, US2003/147842, US6 153 569 et US22003/157049 décrivent des compositions comprenant un tensioactif anionique et une silicone aminée.

La présente invention a pour but de proposer des compositions ne présentant pas les inconvénients des compositions citées ci-dessus.

Les agents conditionneurs doivent également être véhiculés sur les matières kératiniques traitées en vue de leur conférer, suivant l'application, de bonnes propriétés cosmétiques notamment de douceur, de lissage, de brillance et de démêlage.

Ainsi, à la suite d'importantes recherches menées sur la question, il a maintenant été trouvé par la Demanderesse, qu'en utilisant une association de 3 types de tensioactifs et au moins une silicone aminée, il est possible d'obtenir des compositions détergentes stables présentant d'excellentes propriétés cosmétiques, en particulier le démêlage, la légèreté et le lissage des cheveux traités et ayant de bonnes propriétés d'usage tel qu'un bon pouvoir lavant intrinsèque et un bon pouvoir moussant.

La mise en oeuvre industrielle est extrêmement facile et les propriétés cosmétiques des shampooings sont excellentes.

Les compositions obtenues sont stables au stockage, sans nécessiter l'addition d'agent de dispersion et/ou de mise en suspension de la silicone selon l'invention.

En l'absence de composés additionnels insolubles, les compositions obtenues sont également transparentes. Elles peuvent contenir des quantités importantes de silicone aminée tout en conservant une bonne transparence et en ayant de bonnes propriétés cosmétiques.

Les compositions conformes à l'invention présentent de très bonnes qualités d'usage (mousse abondante et aérée se développant rapidement) ainsi qu'une très bonne rinçabilité.

Les compositions conformes à l'invention confèrent aux matières kératiniques notamment les cheveux, un remarquable

effet traitant qui se manifeste notamment par une facilité de démêlage, ainsi qu'un apport de volume, de légèreté, de lissage, de douceur et de souplesse sans aucun effet de charge. Les cheveux ont un aspect naturel, propre et non gras.

Ainsi, la présente invention a pour objet de nouvelles compositions cosmétiques détergentes et conditionnantes caractérisée par le fait qu'elles comprennent, dans un milieu aqueux cosmétiquement acceptable,

(A) au moins un tensioactif anionique sulfate ou sulfonate,
(B) au moins un agent tensioactif anionique carboxylique différent du tensioactif (A),
(C) au moins un tensioactif amphotère et
(D) au moins une silicone aminée,

le rapport en poids tensioactif anionique sulfate ou sulfonate / tensioactif anionique carboxylique allant de 2 à 12,
la quantité totale de tensioactifs représentant de 4 % à 35 % en poids par rapport au poids total de la composition finale.

L'invention a également pour objet l'utilisation en cosmétique des compositions ci-dessus pour le nettoyage et /ou le démaquillage et/ou le conditionnement des matières kératiniques telles que les cheveux et la peau.

(A) Tensioactifs sulfate(s) ou sulfonate(s)

Selon l'invention, les tensioactifs anioniques sulfate(s) ou sulfonate(s) sont des tensioactifs anioniques comportant au moins une fonction sulfate ($-OSO_3H$ ou - $OSO_3^-$) et/ou une fonction sulfonate ($-SO_3H$ ou ($-SO_3^-$).

Les tensioactifs anioniques sulfates ou sulfonates utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Parmi ces tensioactifs anioniques, on préfère utiliser les sels d'alkyléthersulfates en $C_8$-$C_{14}$ et plus particulièrement ceux en $C_{12}$-$C_{14}$. Ces sels comprennent de préférence de 2 à 5 groupements d'oxyde d'éthylène.
On utilise de préférence un agent tensioactif anionique choisi parmi les alkyl($C_{12}$-$C_{14}$) sulfates de sodium, de triéthanolamine, de magnésium ou d'ammonium, les alkyl ($C_{12}$-$C_{14}$)éthersulfates de sodium, d'ammonium ou de magnésium oxyéthylénés à 2,2 moles d'oxyde d'éthylène, le cocoyl iséthionate de sodium, les méthyltaurates.

Les tensioactifs anioniques sulfates ou sulfonates sont généralement présents à raison de 1,5 % à 34,4 % en poids, de préférence de 2 à 25 % en poids, particulièrement de 5 à 25% en poids et plus particulièrement de 8 à 20% en poids par rapport au poids total de la composition.

(B) Tensioactifs anioniques carboxyliques

Selon l'invention, les tensioactifs anioniques carboxyliques sont des tensioactifs anioniques comportant au moins une fonction carboxylique (-COOH) éventuellement sous forme de sel (-COO-).
Les tensioactifs anioniques de type carboxyliques différents des tensioactifs (A) ne comprennent de préférence pas de fonction sulfate ou sulfonate et peuvent être notamment choisis parmi les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés et leurs sels, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène tels que les composés proposés par la société KAO sous les dénominations AKYPO, les acyl($C_6$-$C_{24}$) sarcosinates et leurs sels, les acyl ($C_6$-$C_{24}$)lactylates et leurs sels et les acyl($C_6$-$C_{24}$) glutamates. On peut également utiliser les esters d'alkyl($C_6$-$C_{24}$) polyglycosides carboxyliques tels que les alkylglucoside acétates, les alkylglucoside citrates et les alkylpolyglycoside tartrate. De tels produits sont notamment vendus sous les dénominations de EUCAROL APG/EC et EUCAROL APG/ET par la société LAMBERTI et PLANTAPON LGC SORB par la société COGNIS.
On peut également utiliser les mélanges de ces tensioactifs.
Les sels sont en particulier choisis parmi les sels alcalins, notamment de sodium, les sels d'ammonium, les sels d'amines, les sels d'aminoalcools tels la triéthanolamine ou la monoéthanolamine et les sels de magnésium.

Les tensioactifs anioniques du type acides ou sels d'éthers carboxyliques polyoxyalkylénés sont en particulier ceux qui répondent à la formule (1) suivante :

$$R_1-(OC_2H_4)_n-OCH_2COOA \qquad (1)$$

dans laquelle :

R1 représente un radical ou un mélange de radicaux alkyle ou alcényle linéaire ou ramifié en $C_8$-$C_{22}$, un radical alkyl ($C_8$-$C_9$)phényle, un radical R2CONH-$CH_2$-$CH_2$- avec R2 désignant un radical alkyle ou alcényle linéaire ou ramifié en $C_9$-C21,

et n est un nombre entier ou décimal (valeur moyenne) pouvant varier de 2 à 24 et de préférence de 2 à 10, le radical alkyle ayant entre 6 et 20 atomes de carbone environ, de préférence de 8 à 18 atomes de carbone et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements $R_1$ sont différents.

Les acides éther carboxylique oxyalkylénés ou leurs sels utilisés de préférence selon la présente invention sont choisis parmi ceux de formule (I) dans laquelle R1 désigne un radical ou un mélange de radicaux alkyl($C_{12}$-$C_{14}$), cocoyle, oléyle; un radical nonylphényle ou octylphényle, A désigne un atome d'hydrogène ou de sodium, et n varie de 2 à 20 et de préférence 2 à 10.

On utilise de préférence les acides alkyl ($C_6$-$C_{24}$) éther carboxyliques polyoxyalkylénés et leurs sels, les acides alkyl ($C_6$-$C_{24}$) amido éther carboxyliques polyoxyalkylénés en particulier ceux comportant de 2 à 15 groupements oxyde d'alkylène, et leurs sels et leurs mélanges.

Plus préférentiellement encore, on utilise des composés de formule (I) dans laquelle R désigne un radical alkyl ($C_{12}$), A désigne un atome d'hydrogène ou de sodium, et n varie de 2 à 10.

Parmi les produits commerciaux, on peut utiliser de préférence les produits vendus par la société CHEM Y sous les dénominations :

AKYPO® NP 70 (R=nonylphényle, n=7, p=0, A=H)
AKYPO® NP 40 (R=nonylphényle, n=4, p=0, A=H)
AKYPO® OP 40 (R=octylphényle, n=4, p=0, A=H)
AKYPO® OP 80 (R=octylphényle, n=8, p=0, A=H)
AKYPO® OP 190 (R=octylphényle, n=19, p=0, A=H)
AKYPO® RLM 38 (R= alkyl($C_{12}$-$C_{14}$), n=3,8, p=0, A=H)
AKYPO® RLM 38 NV (R= alkyl($C_{12}$-$C_{14}$), n=4, p=0, A=Na)
AKYPO® RLM 45 (R= alkyl($C_{12}$-$C_{14}$), =4,5, p=0, A=H)
AKYPO® RLM 45 NV (R= alkyl($C_{12}$-$C_{14}$), n=4,5, p=0, A=Na)
AKYPO® RLM 100 (R= alkyl($C_{12}$-$C_{14}$), n=10, p=0, A=H)
AKYPO® RLM 100 NV (R= alkyl($C_{12}$-$C_{14}$), n=10, p=0, A=Na)
AKYPO® RLM 130 (R= alkyl($C_{12}$-$C_{14}$), n=13, p=0, A=H)
AKYPO® RLM 160 NV (R= alkyl($C_{12}$-$C_{14}$), n=16, p=0, A=Na)

ou par la société SANDOZ sous les dénominations :

SANDOPAN DTC-Acid (R= alkyl($C_{13}$), n=6, p=0, A=H)
SANDOPAN DTC (R= alkyl($C_{13}$), n=6, p=0, A=Na)
SANDOPAN LS 24 (R= alkyl($C_{12}$-$C_{14}$), n=12, p=0, A=Na)
SANDOPAN JA 36 (R= alkyl($C_{13}$), n=18, p=0, A=H),

et plus particulièrement, les produits vendus sous les dénominations suivantes :

AKYPO® RLM 45
AKYPO® RLM 100
AKYPO® RLM 38.

On utilise de préférence les acides alkyl ($C_6$-$C_{24}$) éther carboxyliques polyoxyalkylénés et leurs sels, les acides alkyl($C_6$-$C_{24}$) amido éther carboxyliques polyoxyalkylénés et leurs sels en particulier ceux comportant de 2 à 15 groupements oxyde d'alkylène et les esters d'alkyl($C_6$-$C_{24}$)polyglycosides carboxyliques et leurs sels et leurs mélanges.

Les tensioactifs anioniques carboxyliques différents des tensioactifs cités en A) sont généralement présents à raison de 0,5 % à 15 % en poids, de préférence de 1 à 10 % en poids, plus particulièrement de 1 à 5% en poids et

encore plus préférentiellement de 1,5 à 3% en poids par rapport au poids total de la composition.

(C) Tensioactif(s) amphotère(s) et/ou zwittérioniques:

Les agents tensioactifs amphotères et/ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits décrits dans les brevets US-2 528 378 et US-2 781 354 et de structures :

$$R_2\text{-CONHCH}_2\text{CH}_2\text{-N}(R_3)(R_4)(\text{CH}_2\text{COO-}) \qquad (2)$$

dans laquelle : $R_2$ CO désigne un radical acyle en C6-C24, par exemple un radical présent dans l'huile de coprah hydrolysée, un radical octoyle, décoyle ou dodécanoyle et leurs mélanges, $R_3$ désigne un groupement bêta-hydroxyéthyle et

$R_4$ un groupement carboxyméthyle ;

et

$$R_{2'}\text{-CONHCH}_2\text{CH}_2\text{-N(B)(C)} \qquad (3)$$

dans laquelle :

B représente $-CH_2CH_2OX'$, C représente $-(CH_2)_z$ -Y', avec z = 1 ou 2,
X' désigne le groupement $-CH_2CH_2-COOH$ ou un atome d'hydrogène
Y' désigne -COOH ou le radical $-CH_2$ - CHOH - $SO_3H$
$R'_2$ CO désigne un radical acyle en C6-C24, par exemple un radical présent dans l'huile de coprah hydrolysée ou l'huile de lin, un radical octoyle, décoyle ou dodécanoyle, stéaroyle ou isostéaroyle, oléoyle et leurs mélanges.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines, en particulier la cocamidopropylbétaine telles que la TEGOBETAINE® F50 commercialisée par la société GOLDSCHMIDT.

Le ou les tensio-actif(s) amphotère(s) sont généralement présents dans des quantités allant de 0,1 à 20 % en poids, de préférence de 1 à 10 % en poids, et plus particulièrement de 1,5 à 5% en poids par rapport au poids total de la composition.

Ainsi, selon l'invention, la quantité totale de tensioactifs peut représenter de 4 % à 35 % en poids, plus particulièrement de 6 à 25% en poids, de préférence de 8 % à 20 % en poids, du poids total de la composition finale.

Le rapport en poids tensioactif anionique sulfate ou sulfonate / tensioactif amphotère va de préférence de 2 à 12, plus particulièrement de 4 à 10 et encore plus préférentiellement de 5 à 8.

Le rapport en poids tensioactif anionique sulfate ou sulfonate / tensioactif anionique carboxylique va de préférence de 4 à 10.

Le rapport en poids tensioactif anionique carboxylique/ tensioactif amphotère va de préférence de 0,3 à 3 et encore plus préférentiellement de 0,5 à 1,5.

D- Silicone aminée

Les compositions de la présente invention contiennent au moins une silicone aminée. On désigne par silicone aminée toute silicone comportant au moins une amine primaire, secondaire, tertiaire ou un groupement ammonium

quaternaire. On peut ainsi citer :

a) les polysiloxanes répondant à la formule (I):

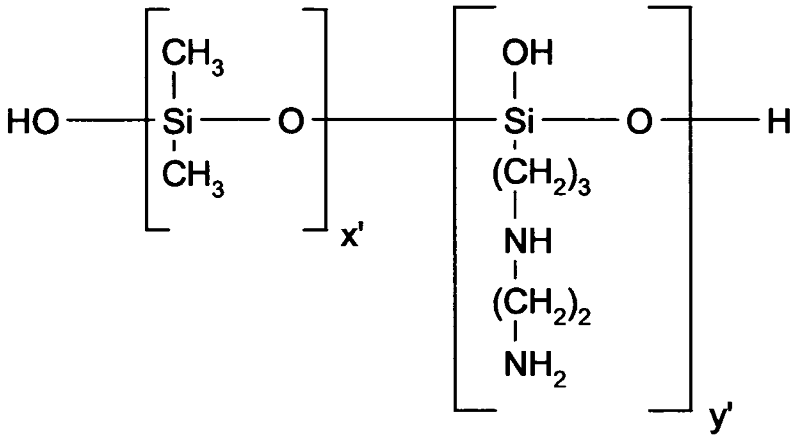

(I)

dans laquelle x' et y' sont des nombres entiers dépendant du poids moléculaire, généralement tels que ledit poids moléculaire moyen en poids est allant de 5 000 et 500 000 environ ;

b) les silicones aminées répondant à la formule:

$$R'_aG_{3-a}\text{-}Si(OSiG_2)_n\text{-}(OSiG_bR'_{2-b})_m\text{-}O\text{-}SiG_{3-a}\text{-}R'_a \qquad (II)$$

dans laquelle :

G, identiques ou différents, désignent un atome d'hydrogène, ou un groupement phényle, OH, alkyle en $C_1$-$C_8$, par exemple méthyle, ou alcoxy en $C_1$-$C_8$, par exemple méthoxy
a identiques ou différents, désignent le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R', identiques ou différents, désignent un radical monovalent de formule - CqH2qL dans laquelle q est un nombre de 2 à 8 et L est un groupement aminé éventuellement quaternisé choisi parmi les groupements:

$-NR''\text{-}Q\text{-}N(R'')_2$

$-N(R'')_2$

$-N^+(R'')_3\ A^-$

$-N^+H(R'')_2\ A^-$

$-N^+H_2(R'')\ A^-$

$-N(R'')\text{-}Q\text{-}N^+R''H_2\ A^-$

$-NR''\text{-}Q\text{-}N^+\ (R'')_2H\ A^-$

$-NR''\text{-}Q\text{-}N^+\ (R'')_3\ A^-$,

dans lesquels R" peut désigner hydrogène, phényle, benzyle, ou un radical hydrocarboné saturé monovalent, par

exemple un radical alkyle ayant de 1 à 20 atomes de carbone ; Q désigne un groupement de formule $C_rH_{2r}$, linéaire ou ramifié, r étant un entier allant de 2 à 6, de préférence de 2 à 4 ; et A-représente un ion halogénure tel que par exemple fluorure, chlorure, bromure
ou iodure.
Un groupe de silicones aminées correspondant à cette définition est représentée par les silicones dénommées "triméthylsilylamodiméthicone", répondant à la formule :

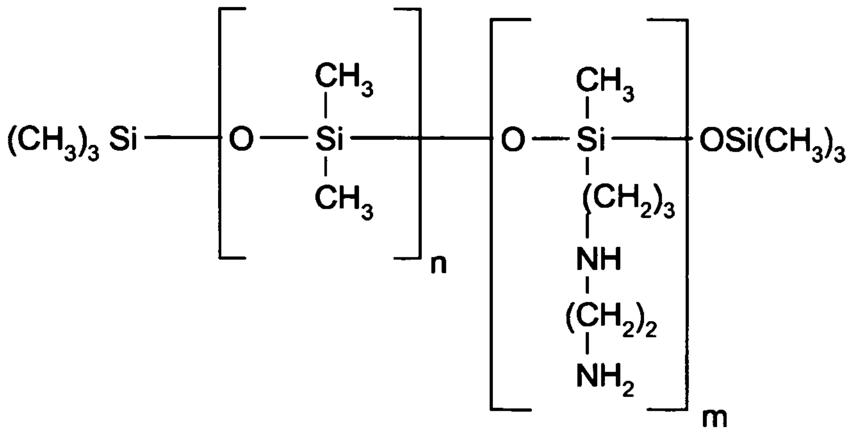

(III)

dans laquelle n et m ont les significations données ci-dessus (cf formule II).
De tels polymères sont décrits par exemple dans la demande de brevet EP-A-95238.
Un autre groupe de silicones aminées correspondant à cette définition est représentée par les silicones de formules (IV) ou (V) suivantes :

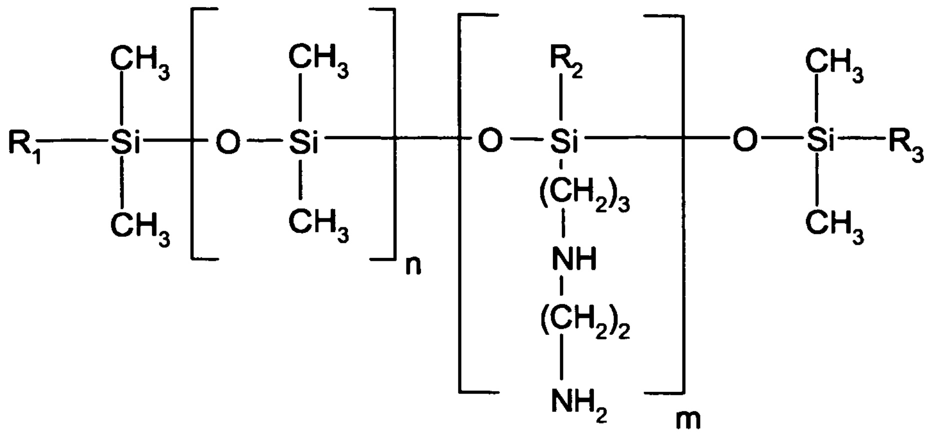

(IV)

dans laquelle :

m et n sont des nombres tels que la somme (n + m) peut varier notamment de 1 à 1 000 et en particulier de 50 à 250 et plus particulièrement de 100 à 200,
n pouvant désigner un nombre de 0 à 999 et notamment de 49 à 249 et plus particulièrement de 125 à 175 et m pouvant désigner un nombre de 1 à 1 000, et notamment de 1 à 10 et plus particulièrement de 1 à 5,
$R_1$, $R_2$, $R_3$, identiques ou différents, représentent un radical hydroxy ou alcoxy en $C_1$-$C_4$, l'un au moins des radicaux $R_1$ à $R_3$ désignant un radical alcoxy.

De préférence le radical alcoxy est un radical méthoxy.
Le rapport molaire Hydroxy/Alcoxy va de préférence de 0,2:1 à 0,4:1 et de préférence de 0,25:1 à 0,35:1 et plus particulièrement est égal à 0,3:1.

La masse moléculaire moyenne en poids de la silicone va de préférence de 2000 à 1000000 et encore plus particulièrement de 3500 à 200000.

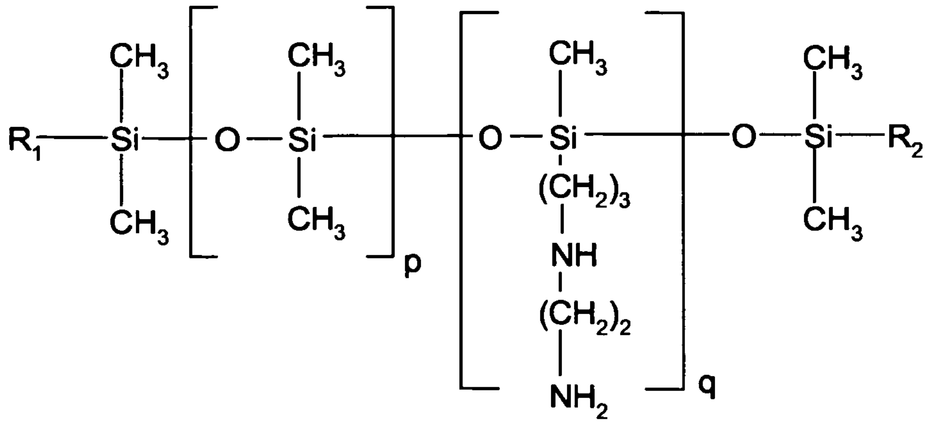

(V)

dans laquelle :

p et q sont des nombres tels que la somme (p + q) peut varier notamment de 1 à 1 000 et en particulier de 50 à 350, et plus particulièrement de 150 à 250,
p pouvant désigner un nombre de 0 à 999 et notamment de 49 à 349 et plus particulièrement de 159 à 239 et q pouvant désigner un nombre de 1 à 1 000, et notamment de 1 à 10 et plus particulièrement de 1 à 5;
$R_1$, $R_2$, différents, représentent un radical hydroxy ou alcoxy en $C_1$-$C_4$, l'un au moins des radicaux $R_1$ ou $R_2$ désignant un radical alcoxy.

De préférence le radical alcoxy est un radical méthoxy. Le rapport molaire hydroxy/Alcoxy va généralement de 1: 0,8 à 1:1,1 et de préférence de 1:0,9 à 1:1 et plus particulièrement est égal à 1:0,95. La masse moléculaire moyenne en poids de la silicone va de préférence de 2000 à 200000 et encore plus particulièrement de 5000 à 100000 et plus particulièrement de 10000 à 50000.
Les masses moléculaires moyennes en poids de ces silicones aminées sont mesurées par Chromatographie par Perméation de Gel (GPC) à température ambiante en équivalent polystyrène. Les colonnes utilisées sont des colonnes $\mu$ styragel. L'éluant est le THF, le débit est de 1 ml/mn. On injecte 200 $\mu$l d'une solution à 0,5% en poids de silicone dans le THF. La détection se fait par réfractométrie et UVmétrie
Les produits commerciaux correspondant à ces silicones de structure (IV) ou (V) peuvent inclure dans leur composition une ou plusieurs autres silicones aminées dont la structure est différente des formules (IV) ou (V).
Un produit contenant des silicones aminées de structure (IV) est proposé par la société WACKER sous la dénomination BELSIL® ADM 652.
Un produit contenant des silicones aminées de structure (V) est proposé par WACKER sous la dénomination Fluid WR 1300®.
Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs.
Les tensioactifs peuvent être de toute nature mais de préférence cationique et/ou non ionique.
La taille moyenne en nombre des particules de silicone dans l'émulsion va généralement de 3 nm à 500 nanomètres .
De préférence, notamment comme silicones aminées de formule (V), on utilise des microémulsions dont la taille moyenne des particule va de 5 nm à 60 nanomètres (bornes incluses) et plus particulièrement de 10 nm à 50 nanomètres (bornes incluses).
Ainsi, on peut utiliser selon l'invention les microémulsions de silicone aminée de formule (V) proposées sous les dénominations FINISH CT 96 E® ou SLM 28020® par la société WACKER.
De préférence la silicone aminée est choisie de façon telle que l'angle de contact avec l'eau d'un cheveu traité avec une composition contenant 2% (matière active) de ladite silicone selon l'invention varie de 90° à 180° (bornes incluses) et de préférence de 90 à 130° (bornes incluses).
Pour mesurer l'angle de contact, la silicone aminée est de préférence solubilisée ou dispersée dans un solvant de la silicone aminée ou de l'émulsion de silicone aminée (notamment l'hexaméthyldisiloxane ou l'eau selon l'hydrophilie

de la silicone).
De préférence la composition contenant la ou les silicones aminées de formule (IV) ou (V) est telle que l'angle de contact avec l'eau d'un cheveu traité avec ladite composition varie de 90 à 180°(bornes incluses) et de préférence de 90 à 130° (bornes incluses).
La mesure de l'angle de contact est basée sur l'immersion d'un cheveu dans de l'eau distillée. Il consiste à évaluer la force exercée par l'eau sur le cheveu lors de son immersion de l'eau distillée et lors de son retrait. Les forces ainsi mesurées sont directement reliées à l'angle de contact θ entre l'eau et la surface du cheveu. Le cheveu est dit hydrophile lorsque l'angle θ est situé de 0 à 90° (bornes incluses), hydrophobe lorsque cet angle est situé de 90° à 180° (bornes incluses).
Le test s'effectue avec des mèches de cheveux naturels ayant été décolorés dans les mêmes conditions puis lavés. Chaque mèche de 1 g est placée dans un cristallisoir de 75 mm de diamètre puis recouverte de façon homogène par 5 mL de la formule à tester. La mèche est ainsi laissée 15 minutes à température ambiante puis rincée à l'eau distillée pendant 30 secondes. La mèche essorée est laissée à l'air libre jusqu'à ce qu'elle soit complètement sèche.
Pour chaque évaluation, 10 cheveux ayant subi le même traitement sont analysés. Chaque échantillon, fixé à une microbalance de précision, est immergé par la pointe dans un récipient rempli d'eau distillée. Cette balance DCA (« Dynamic Contact Angle Analyser »), de la société CAHN Instruments, permet de mesurer la force (F) exercée par l'eau sur le cheveu.
Parallèlement, le périmètre du cheveu (P) est mesuré *via* une observation microscopique.
La force moyenne de mouillabilité sur 10 cheveux et la section des cheveux analysés permettent d'obtenir l'angle de contact du cheveu sur l'eau, selon la formule :

$$F = P * \Gamma lv * \cos\theta$$

où F est la force de mouillabilité exprimée en Newton, P le périmètre du cheveu en mètre, Γlv la tension interfaciale liquide / vapeur de l'eau en $J/m^2$ et θ l'angle de contact.
Le produit SLM 28020 de WACKER à 12% dans l'eau (soit 2% en silicone aminée) conduit à un angle de contact de 93° selon le test indiqué ci-dessus.
Le produit BELSIL ADM 652 de WACKER à 2% dans l'hexaméthyldisiloxane (soit 2% en silicone aminée) conduit à un angle de contact de 111° selon le test indiqué ci-dessus.
Un autre groupe de silicones aminées correspondant à cette définition est représenté par la formule suivante (VI):

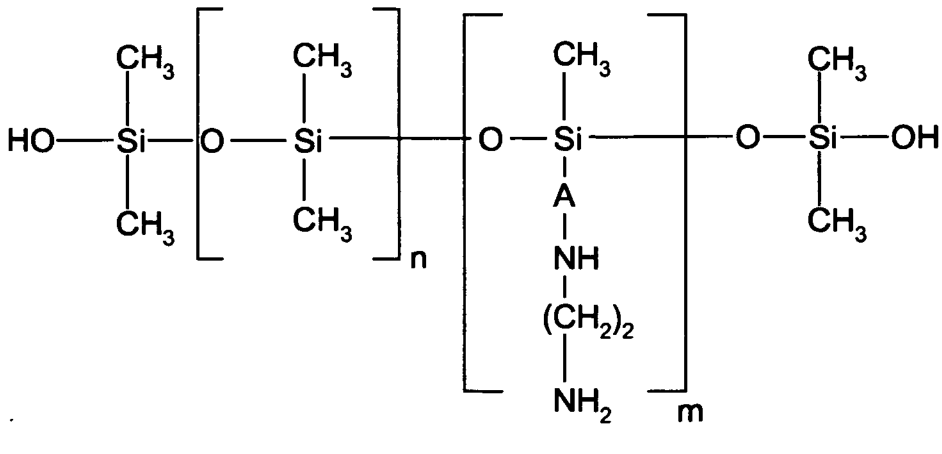

(VI)

dans laquelle :

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
A désigne un radical alkylène linéaire ou ramifié ayant de 4 à 8 atomes de carbone et de préférence 4 atomes

de carbone. Ce radical est de préférence linéaire.

La masse moléculaire moyenne en poids des silicones aminées va de préférence de 2000 à 1000000 et encore plus particulièrement de 3500 à 200.000.
Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.
Selon l'invention la viscosité à 25°C de la silicone aminée est notamment supérieure à 25000 cSt ($mm^2/s$) et de préférence va de 30000 à 200000 cSt ($mm^2/s$). et plus particulièrement de 30000 à 150000 cSt ($mm^2/s$).
Ces silicones aminées, ont de préférence un indice d'amine inférieur ou égal à 0,4 meq./g, de préférence allant de 0,001 à 0,2 meq./g, et plus particulièrement allant de 0,01 à 0,1 meq./g.
L'indice d'amine est le nombre de milli-équivalents amine par gramme de composé. Cet indice est déterminé de façon tout à fait classique par des méthodes de titrage par indicateur coloré ou par titrage potentiométrique.
Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs.
Les tensioactifs peuvent être de toute nature mais de préférence cationiques et/ou non ioniques.
La taille moyenne en nombre des particules de silicone dans l'émulsion va généralement de 3 nm à 500 nanomètres, de préférence de 5 nm à 300 nanomètres et plus particulièrement de 10 nm à 275 nanomètres et encore plus préférentiellement de 150 à 275 nanomètres.
Une silicone répondant à cette formule est par exemple la DC2-8299 CATIONIC EMULSION de DOW CORNING.
Un autre groupe de silicones aminées correspondant à cette définition est représenté par la formule suivante (VII):

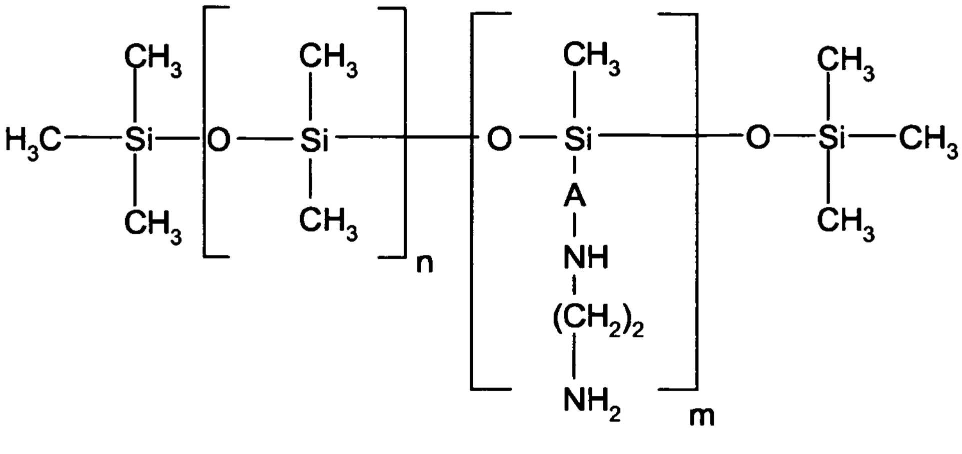

(VII)

dans laquelle :

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
A désigne un radical alkylène linéaire ou ramifié ayant de 4 à 8 atomes de carbone et de préférence 4 atomes de carbone. Ce radical est de préférence ramifié.

La masse moléculaire moyenne en poids des silicones aminées va de préférence de 500 à 1000000 et encore plus particulièrement de 1000 à 200.000.
Les masses moléculaires moyennes en poids de ces silicones aminées sont mesurées par Chromatographie par Perméation de Gel (GPC) à température ambiante en équivalent polystyrène. Les colonnes utilisées sont des colonnes μ styragel. L'éluant est le THF, le débit est de 1 ml/mn. On injecte 200 μl d'une solution à 0,5% en poids de silicone dans le THF. La détection se fait par réfractométrie et UVmétrie.
Selon l'invention la viscosité à 25°C de la silicone aminée est notamment supérieure à 500 cSt ($mm^2/s$) et de

préférence va de 1000 à 200000 cSt ($mm^2/s$). et plus particulièrement de 1500 à 10000 cSt ($mm^2/s$).

Ces silicones aminées, ont de préférence un indice d'amine supérieur à 0,4 meq./g, de préférence allant de 0,5 à 3 meq./g, et plus particulièrement allant de 0,5 à 1 meq./g.

L'indice d'amine est le nombre de milli-équivalents amine par gramme de composé. Cet indice est déterminé de façon tout à fait classique par des méthodes de titrage par indicateur coloré ou par titrage potentiométrique.

Lorsque ces silicones aminées sont mises en oeuvre, une forme de réalisation particulièrement intéressante est leur utilisation sous forme d'émulsion huile dans eau. L'émulsion huile dans eau peut comprendre un ou plusieurs tensioactifs.

Les tensioactifs peuvent être de toute nature mais de préférence cationiques et/ou non ioniques.

La taille moyenne en nombre des particules de silicone dans l'émulsion va généralement de 3 nm à 500 nanomètres, de préférence de 5 nm à 300 nanomètres et plus particulièrement de 10 nm à 275 nanomètres et encore plus préférentiellement de 150 à 275 nanomètres.

Une silicone répondant à cette formule est par exemple la DC2-8566 Amino fluid de DOW CORNING.

c) les silicones aminées répondant à la formule (VIII) :

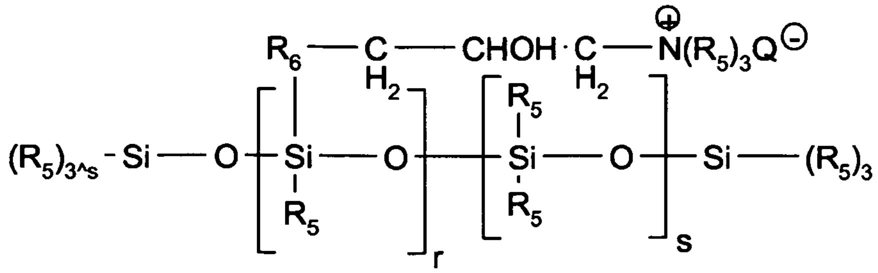

(VIII)

dans laquelle :

$R_5$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle ;

$R_6$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ relié au Si par une liaison SiC;

Q- est un anion tel qu'un ion halogénure, notamment chlorure ou un sel d'acide organique (acétate ...);

r représente une valeur statistique moyenne de 2 à 20 et en particulier de 2 à 8 ; s représente une valeur statistique moyenne de 20 à 200 et en particulier de 20 à 50.

De telles silicones aminées sont décrites plus particulièrement dans le brevet US 4 185 087.

d) les silicones ammonium quaternaire de formule:

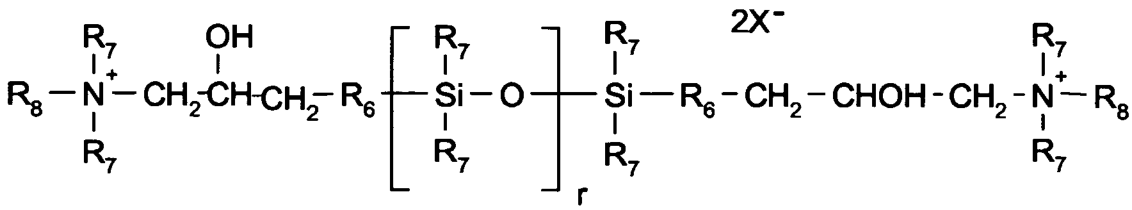

(IX)

dans laquelle :

$R_7$, identiques ou différents, représentent un radical hydrocarboné monovalent ayant de 1 à 18 atomes de carbone, et en particulier un radical alkyle en $C_1$-$C_{18}$, un radical alcényle en $C_2$-$C_{18}$ ou un cycle comprenant 5 ou 6 atomes de carbone, par exemple méthyle ;

$R_6$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ relié au Si par une liaison SiC ;
$R_8$, identiques ou différents, représentent un atome d'hydrogène, un radical hydrocarboné monovalent ayant de 1 à 18 atomes de carbone, et en particulier un radical alkyle en $C_1$-$C_{18}$, un radical alcényle en $C_2$-$C_{18}$, un radical -$R_6$-$NHCOR_7$;
X- est un anion tel qu'un ion halogénure, notamment chlorure ou un sel d'acide organique (acétate ...);
r représente une valeur statistique moyenne de 2 à 200 et en particulier de 5 à 100 ;

Ces silicones sont par exemple décrites dans la demande EP-A-0530974.

e) les silicones aminées de formule :

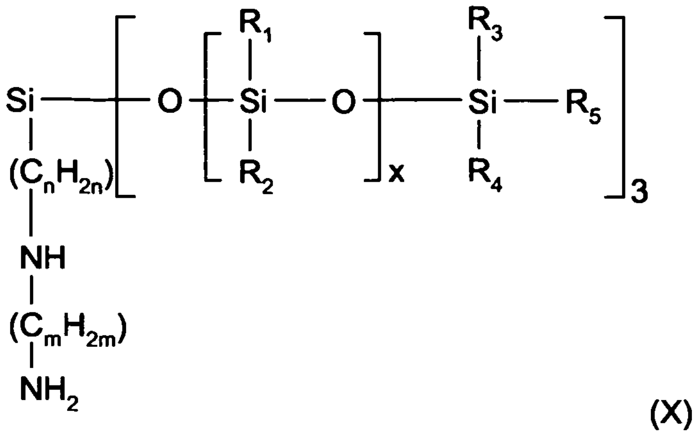

(X)

dans laquelle :

- $R_1$, $R_2$, $R_3$ et $R_4$, identiques ou différents, désignent un radical alkyle en $C_1$-$C_4$ ou un groupement phényle,
- $R_5$ désigne un radical alkyle en $C_1$-$C_4$ ou un groupement hydroxyle,
- n est un entier variant de 1 à 5,
- m est un entier variant de 1 à 5,

et dans laquelle x est choisi de manière telle que l'indice d'amine varie de 0,01 à 1 meq/g.

f) les silicones aminées polyoxyalkylénées de type $(XY)_i$ , X étant un bloc polysiloxane et Y étant un bloc polyoxyalkyléné comportant au moins un groupement amine pouvant, de préférence, être constituées d'unités répétitives de formule générale suivante:

$$[SiMe_2\text{-}O\text{- }(SiMe_2O)_xSiMe_2\text{ - }R\text{-}N(H)\text{-}R'\text{-}O\text{ -}(C_2H_4O)_a\text{ - }(C_3H_6O)_b\text{ - }R'\text{-}N(H)\text{-}R\text{-}] \qquad (XI)$$

dans laquelle:

- a est un nombre entier supérieur ou égal à 1, de préférence allant de 5 à 200 et encore plus particulièrement de 5 à 100 ;
- b est un nombre entier allant de 0 à 200, de préférence allant de 4 à 200 et encore plus particulièrement de 5 à 100 ;
- x est un nombre entier allant de 1 à 10000 et encore plus particulièrement de 10 à 5000 ;
- R, identiques ou différents, représentent un groupe organique divalent qui est lié à l'atome de silicium adjacent par une liaison carbone-silicium et à un atome d'azote,
- R', identiques ou différents, représentent un groupe organique divalent qui est lié à l'atome d'oxygène adjacent par une liaison carbone-oxygène et à un atome d'azote,
R est de préférence un radical hydrocarboné en $C_2$-$C_{12}$ comportant éventuellement un ou plusieurs hétéroatomes tels que l'oxygène. Plus particulièrement, R désigne un radical éthylène, propylène linéaire ou ramifié, butylène linéaire ou ramifié ou -$CH_2CH_2CH_2OCH(OH)CH_2$-.
R' est de préférence un radical hydrocarboné en $C_2$-$C_{12}$ comportant éventuellement un ou plusieurs hétéroatomes tels que l'oxygène. Plus particulièrement R' désigne un radical alkylène divalent comme par exemple

l'éthylène, le propylène linéaire ou ramifié ou le butylène linéaire ou ramifié.

Les blocs siloxane représentent généralement de 50 à 95% en moles par rapport au poids total de la silicone et plus particulièrement de 70 à 85% en moles.

Le taux d'amine va généralement de 0,02 à 0,5 meq/g de copolymère dans une solution à 30% dans le dipropylèneglycol et plus particulièrement de 0,05 à 0,2.

Le poids moléculaire moyen en poids de la silicone de formule (XI) est de préférence allant de 5000 à 1000000 et encore plus particulièrement de 10000 à 200000.

La silicone particulièrement visée par cette formule (XI) est celle commercialisée sous la marque Silsoft A-843 Organosilicone Copolymer par OSI. Les silicones aminées particulièrement préférées conformément à l'invention sont celles de formule (I) et (II) et leurs mélanges.

Les silicones aminées particulièrement préférées conformément à l'invention sont celles de formule (I) à (VII) et plus particulièrement celles de formule (I), (IV), (V), (VI), (VII).

La ou les silicones aminées sont utilisées de préférence en une quantité allant de 0,01 à 10% en poids par rapport au poids total de la composition. Plus préférentiellement, cette quantité va de 0,1 à 5% en poids et encore plus particulièrement de 0,5 à 3 % en poids par rapport au poids total de la composition.

Selon un mode de réalisation de l'invention, les compositions peuvent comprendre en outre un sel hydrosoluble et/ou un alcool hydrosoluble mono ou polyhydroxylé Les sels hydrosolubles selon l'invention sont de préférence les sels de métaux mono ou divalents et d'un acide minéral ou organique.

On peut citer en particulier le chlorure de sodium, le chlorure de potassium, le chlorure de calcium, le sulfate de magnésium, le citrate de sodium, les sels de sodium de l'acide phosphorique. Le chlorure de sodium est particulièrement préféré.

Les compositions détergentes selon l'invention présentent un pH final généralement compris entre 3 et 8. De préférence, ce pH est compris entre 4 et 7,5. L'ajustement du pH à la valeur désirée peut se faire classiquement par ajout d'une base (organique ou minérale) dans la composition, par exemple de la soude, de l'ammoniaque ou une (poly) amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence l'acide citrique ou l'acide chlorhydrique.

Le milieu aqueux cosmétiquement acceptable peut être constitué uniquement par de l'eau ou par un mélange d'eau et d'un solvant cosmétiquement acceptable. tel qu'un alcool inférieur en $C_1$-$C_4$, comme l'éthanol, l'isopropanol, le tertiobutanol, le n-butanol ; les alkylèneglycols comme le propylèneglycol, l'hexylèneglycol ou le glycérol.

La composition selon l'invention comprend de préférence au moins 30% en poids d'eau et plus particulièrement de 50 à 90% en poids et encore préférentiellement de 70 à 85% en poids par rapport au poids total de la composition.

La composition comprend de préférence moins de 20% en poids de phase grasse, par rapport au poids total de la composition.

La phase grasse comprend tous les corps gras insoluble dans l'eau à température ambiante de la composition tels que notamment les esters gras, les huiles végétales, minérales, synthétiques, les alcools gras, les acides gras, les amides gras, les cires, les silicones. La phase grasse représente de préférence de 0,1 à 15% en poids et plus particulièrement de 0,5 à 10% en poids par rapport au poids total de la composition et encore plus particulièrement de 0,5 à 8% en poids.

Les compositions conformes à l'invention peuvent contenir en plus de l'association définie ci-dessus des agents régulateurs de viscosité tels que des agents épaississants. On peut citer en particulier les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en $C_{10}$-$C_{30}$ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras, les palmitates de sodium ou de magnésium, les stéarates et hydroxystéarates de sodium ou de magnésium, les alcool gras, les dérivés acylés à chaîne grasse tels que les distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou cationiques ou amphotères, les protéines, les hydrolysats de protéines, les céramides, les pseudocéramides, les huiles végétales, les acides gras notamment à chaînes linéaires ou ramifiées en $C_{16}$-$C_{40}$ tels que l'acide méthyl-18 eicosanoïque, les hydroxyacides, les vitamines, les provitamines telle que le panthénol, les silicones, volatiles ou non volatiles, solubles et insolubles dans le milieu différentes des silicones aminées, les filtres UV, les agents hydratants, les agents antipelliculaires ou antiséborrhéiques, les agents antichute des cheveux, les agents anti-radicaux libres, et leurs mélanges.

Selon un mode particulièrement préféré, les compositions selon l'invention comprennent en outre au moins un polymère cationique.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Les polymères cationiques utilisables selon l'invention ont de préférence une densité de charge cationique supérieure ou égale à 0,01 meq./g, et plus particulièrement allant de 0,1 à 3,5 meq./g.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

Selon l'invention, le ou les polymères cationiques peuvent représenter de 0,005 % à 10 % en poids, de préférence de 0,01 % à 5 % en poids, et encore plus préférentiellement de 0,1 % à 3 % en poids, du poids total de la composition finale.

Les compositions selon l'invention peuvent contenir également des synergistes de mousses tels que des 1,2-alcanediols en $C_{10}$-$C_{18}$ ou des alcanolamides gras en $C_{10}$-$C_{18}$ dérivés de mono ou de diéthanolamine.

Bien entendu, l'homme du métier veillera à choisir ce ou ces éventuels composés complémentaires et/ou leurs quantités de manière telle que la solubilité des silicones aminées selon l'invention, la stabilité des composition et les propriétés cosmétiques attachées intrinsèquement à la composition conforme à l'invention ne soient pas, ou substantiellement pas, altérées par la ou les adjonctions envisagées. L'addition de certains composés tels que les agents nacrants peut rendre la composition non transparente.

La transparence peut se mesurer par mesure de la transmittance à 700nm via un spectromètre (par exemple spectromètre Lambda 14 de Perkin Elmer ou UV21 01 PC de Shimadzu) . Les compositions transparentes ont une transmittance supérieure ou égale à 94%, de préférence de 96 à 100%.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :

> La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, et la mousse générée par massage ou friction avec les mains est ensuite éliminée après un éventuel temps de pause, par rinçage avec de l'eau, l'opération pouvant être répétée une ou plusieurs fois.
Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.
Des exemples concrets, illustrant l'invention vont maintenant être donnés.

## EXEMPLES 1 à 3

On a réalisé les compositions de shampooing conformes à l'invention

| | 1 | 2 | 3 |
|---|---|---|---|
| - Lauryléthersulfate de sodium (C12/C14 à 70/30) à 2,2 moles d'oxyde d'éthylène en solution aqueuse à 70% de MA | 14,2 gMA | 14,2 gMA | 10 gMA |
| N-méthyl N-cocoyl taurate de sodium à 30% de MA dans l'eau (HOSTAPON LT-SF de CLARIANT) | — | - | 3 gMA |
| - Cocoylbétaïne à 30% MA (DEHYTON AB 30) | 1,9 gMA | 1,9 gMA | - |
| Disodium cocoamphodiacetate à 39% de MA dans l'eau (MIRANOL C2M CONC. De RHODIA CHIMIE) | - | - | 3 gMA |
| - Acide lauryléther carboxylique à 90% MA dans l'eau (AKYPO RLM 45 CA de KAO) | 1,8 gMA | 1,8 gMA | — |
| - Cocoyl glutamate de triéthanolamine en solution aqueuse à 30% de MA (AMISOFT CT12 de AJINOMOTO) | - | - | 2 gMA |
| - Silicone aminée (DC2-8566 AMINO FLUID de DOW CORNING) | 1 g | | 1 g |
| - Silicone aminée (DC2-8299 CATIONIC EMULSION de DOW CORNING) | | 0,5 g MA | |
| - Cellulose cationique (JR400 de AMERCHOL) | 1 g | 1 g | 1 g |
| - Monoisopropanolamide d'acide de coprah | 3,3 g | 3,3 g | 3,3 g |
| - Parfum, conservateur | qs | qs | qs |
| - Acide chlorhydrique qs pH | 5-5,6 | 5-5,6 | 5-5,6 |
| - Eau déminéralisée qsp | 100 g | 100 g | 100 g |
| | | | |
| Transmittance à 700 nm | >94% | >94% | >94% |

Les compositions selon l'invention sont transparentes et stables. Lorsqu'elles sont appliquées sur les cheveux humides, la mousse est abondante et aérée, elle démarre aisément et se rince facilement et rapidement.
Les cheveux traités avec ces compositions se démêlent facilement et sont légers et lisses de la racine à la pointe.

### Revendications

1. Composition cosmétique détergente et conditionnante, **caractérisée par le fait qu'**elle comprend, dans un milieu aqueux cosmétiquement acceptable, (A) au moins un tensioactif anionique sulfate ou sulfonate, (B) au moins un agent tensioactif anionique carboxylique différent du tensioactif (A), (C) au moins un tensioactif amphotère et (D) au moins une silicone aminée,
le rapport en poids tensioactif anionique sulfate ou sulfonate / tensioactif anionique carboxylique allant de 2 à 12, la quantité totale de tensioactifs représentant de 4 % à 35 % en poids par rapport au poids total de la composition finale.

2. Composition selon l'une quelconque des revendications 1, **caractérisée en ce que** le(s) tensioactif(s) anionique(s) sulfate ou sulfonate est (sont) présent(s) dans des concentrations allant de 1,5 à 34,4 % en poids, de préférence de 2 à 25 % en poids, par rapport au poids total de la composition.

**3.** Composition selon l'une quelconque des revendications 1 à 2, **caractérisée en ce que** le(s) tensioactif(s) amphotère (s) est(sont) présent(s) dans des concentrations allant de 0,1 à 20 % en poids, de préférence de 1 à 10 % en poids, par rapport au poids total de la composition.

**4.** Composition selon l'une quelconque des revendications 1 à 3, **caractérisée en ce que** ledit(s) tensioactif(s) anionique(s) carboxylique(s) est(sont) présent(s) dans des concentrations allant de 0,5 % à 15 % en poids, de préférence de 1 à 10 % en poids par rapport au poids total de la composition.

**5.** Composition selon l'une quelconque des revendications 1 à 4, **caractérisée par le fait que** le rapport en poids tensioactif anionique sulfate ou sulfonate / tensioactif amphotère va de 2 à 12, plus particulièrement de 4 à 10 et encore plus préférentiellement de 5 à 8.

**6.** Composition selon l'une quelconque des revendications 1 à 5, **caractérisée par le fait que** le rapport en poids tensioactif anionique sulfate ou sulfonate / tensioactif anionique carboxylique va de 4 à 10.

**7.** Composition selon l'une quelconque des revendications 1 à 6, **caractérisée par le fait que** le rapport en poids tensioactif anionique carboxylique/ tensioactif amphotère va de 0,3 à 3 et encore plus préférentiellement de 0,5 à 1,5.

**8.** Composition selon l'une quelconque des revendications 1 à 7, **caractérisée par le fait que** la silicone aminée est choisie parmi :

a) les polysiloxanes répondant à la formule (I) :

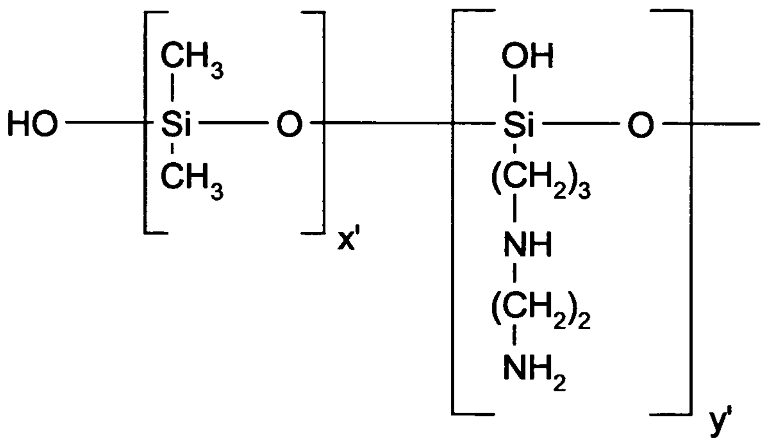

(I)

dans laquelle x' et y' sont des nombres entiers tels que ledit poids moléculaire moyen en poids va de 5 000 à 500 000;

b) les silicones aminées répondant à la formule :

$$R'_aG_{3-a}\text{-Si}(OSiG_2)_n\text{-}(OSiG_bR'_{2-b})_m\text{-O-}SiG_{3-a}\text{-}R'_a \qquad \text{(II)}$$

dans laquelle :

G, identiques ou différents, désignent un atome d'hydrogène, ou un groupement phényle, OH, alkyle en $C_1$-$C_8$, ou alcoxy en $C_1$-$C_8$,

a, identiques ou différents, désignent le nombre 0 ou un nombre entier de 1 à 3, b, désigne 0 ou 1,

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;

R', identiques ou différents, désignent un radical monovalent de formule - CqH2qL dans laquelle q est un

nombre de 2 à 8 et L est un groupement aminé éventuellement quaternisé choisi parmi les groupements :

**$-NR''-Q-N(R'')_2$**
**$-N(R'')_2$**
**$-N^+(R'')_3$ A-**
**$-N^+H(R'')_2$ A-**
**$-N^+H_2(R'')$ A-**
**$-N(R'')-Q-N^+R''H_2$ A-**
**$-NR''-Q-N^+(R'')_2H$ A-**
**$-NR''-Q-N^+(R'')_3$ A-,**

dans lesquels R" peut désigner hydrogène, phényle, benzyle, ou un radical hydrocarboné saturé monovalent, Q désigne un groupement de formule $C_rH_{2r}$, linéaire ou ramifié, r étant un entier allant de 2 à 6, de préférence de 2 à 4 ; et A- représente un ion halogénure,

c) les silicones aminées répondant à la formule (VIII) :

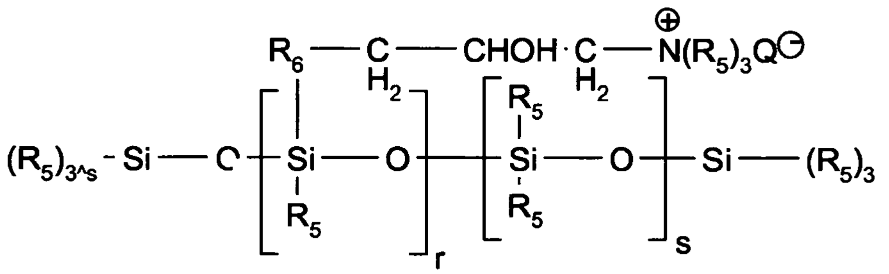

(....)

dans laquelle :

$R_5$ représente un radical hydrocarboné monovalent ayant de 1 à 18 atomes de carbone,
$R_6$ représente un radical hydrocarboné divalent relié au Si par une liaison SiC;
Q- est un anion tel qu'un ion halogénure ou un sel d'acide organique ;
r représente une valeur statistique moyenne de 2 à 20 ;
s représente une valeur statistique moyenne de 20 à 200 ;

d) les silicones ammonium quaternaire de formule (IX) :

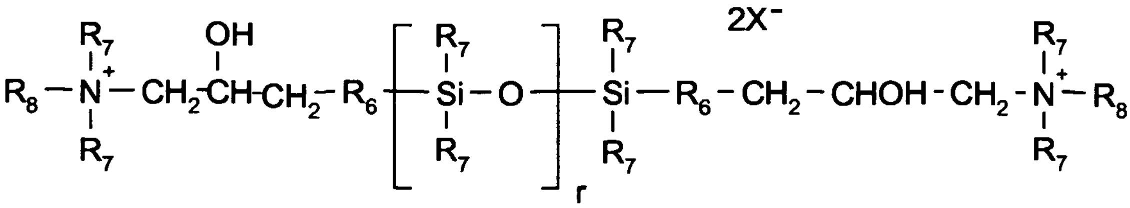

(IX)

dans laquelle :

$R_7$, identiques ou différents, représentent un radical hydrocarboné monovalent ayant de 1 à 18 atomes de carbone ;
$R_6$ représente un radical hydrocarboné divalent relié au Si par une liaison SiC;
$R_8$, identiques ou différents, représentent un atome d'hydrogène, un radical hydrocarboné monovalent ayant de 1 à 18 atomes de carbone;
X- est un anion tel qu'un ion halogénure ou un sel d'acide organique ;
r représente une valeur statistique moyenne de 2 à 200 ;

e) les silicones aminées de formule (X) :

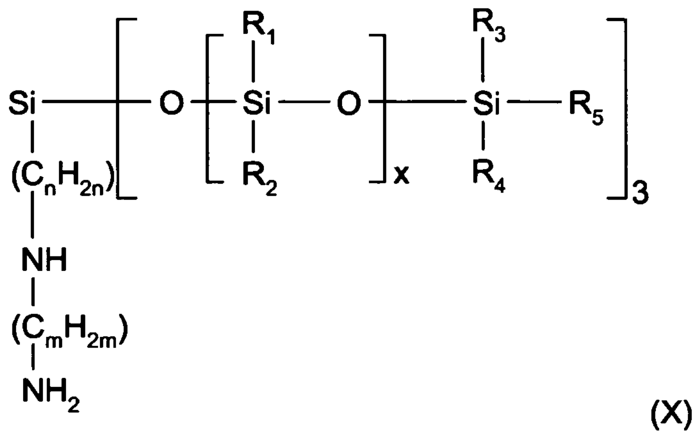

(X)

dans laquelle :

- $R_1$, $R_2$, $R_3$ et $R_4$, identiques ou différents, désignent un radical alkyle en $C_1$-$C_4$ ou un groupement phényle,
- $R_5$ désigne un radical alkyle en $C_1$-$C_4$ ou un groupement hydroxyle,
- n est un entier variant de 1 à 5,
- m est un entier variant de 1 à 5,

et dans laquelle x est choisi de manière telle que l'indice d'amine aille de 0,01 à 1 meq/g;

f) les silicones aminées polyoxyalkylénées de type (XY)i , X étant un bloc polysiloxane et Y étant un bloc polyoxyalkyléné comportant au moins un groupement amine pouvant, de préférence, être constituées d'unités répétitives de formule générale suivante:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \quad (XI)$$

dans laquelle :

- a est un nombre entier supérieur ou égal à 1, de préférence allant de 5 à 200 et encore plus particulièrement de 5 à 100.
- b est un nombre entier allant de 0 à 200, de préférence allant de 4 à 200 et encore plus particulièrement de 5 à 100
- x est un nombre entier allant de 1 à 10000 et encore plus particulièrement de 10 à 5000 ;
- R, identiques ou différents, représentent un groupe organique divalent qui est lié à l'atome de silicium adjacent par une liaison carbone-silicium et à un atome d'azote.
- R', identiques ou différents, représentent un groupe organique divalent qui est lié à l'atome d'oxygène adjacent par une liaison carbone-oxygène et à un atome d'azote.

**9.** Composition selon la revendication précédente, **caractérisée par le fait que** la silicone aminée est choisie parmi les silicones aminées de formule (I) et (II) et leurs mélanges.

**10.** Composition selon l'une quelconques des revendications précédentes, **caractérisée par le fait que** la silicone aminée est choisie parmi les silicones de formule (VI) et de formule (VII).

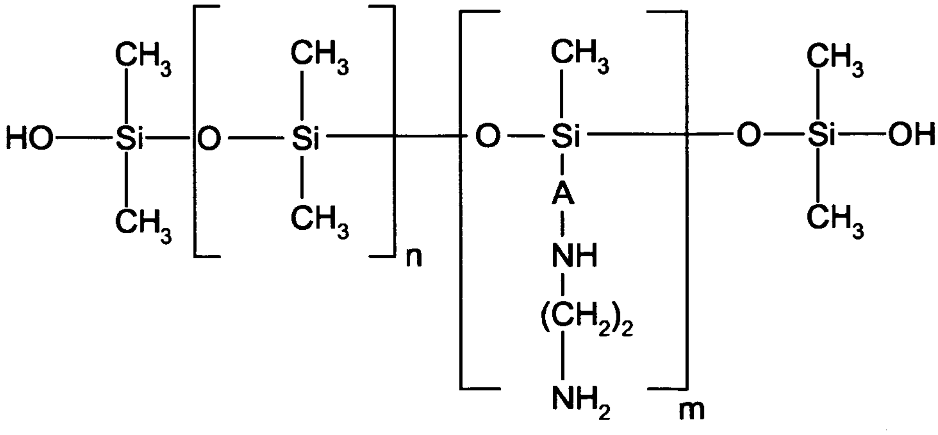

(VI)

dans laquelle :

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;

A désigne un radical alkylène linéaire ou ramifié ayant de 4 à 8 atomes de carbone et de préférence 4 atomes de carbone,

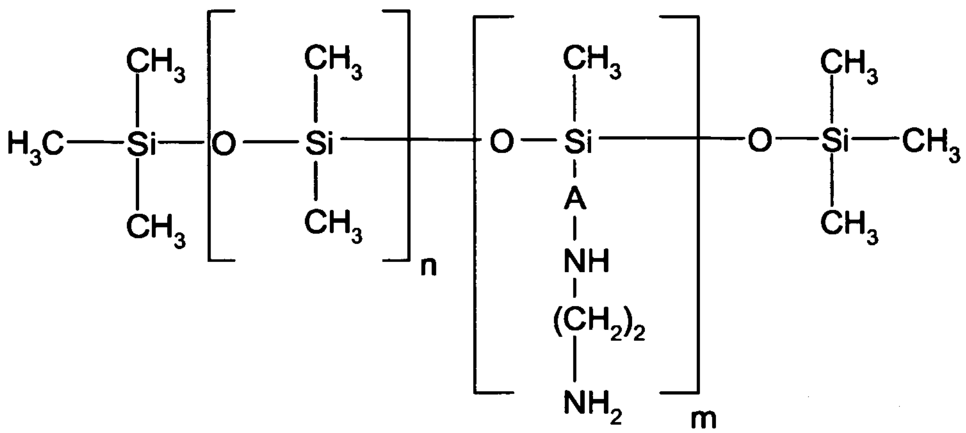

(VII)

dans laquelle :

m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;

A désigne un radical alkylène linéaire ou ramifié ayant de 4 à 8 atomes de carbone et de préférence 4 atomes de carbone.

**11.** Composition selon l'une quelconque des revendications 1 à 10, **caractérisée par le fait que** lesdites silicones aminées sont présentes dans des concentrations allant de 0,01 à 10 %, et de préférence de 0,1 à 5 % en poids par rapport au poids total de la composition et encore plus particulièrement de 0,5 à 3% en poids.

**12.** Composition selon l'une quelconque des revendications 1 à 11, **caractérisée par le fait que** la composition comprend en outre au moins un polymère cationique.

**13.** Composition selon la revendication 123, **caractérisée par** le fait le polymère cationique est choisi parmi les dérivés d'éther de cellulose quaternaires, les copolymères de sel de diallyldiméthylammonium et d'acrylamide, les polysaccharides cationiques, les copolymères quaternaires de vinylpyrrolidone et de sel de vinyl imidazole.

**14.** Composition selon l'une quelconque des revendications 12 à 13, **caractérisée en ce que** ledit polymère cationique représente de 0,005 % à 10 % en poids, de préférence de 0,01 % à 5 % en poids, et encore plus préférentiellement de 0,1 % à 3 % en poids, du poids total de la composition finale.

**15.** Composition selon l'une quelconque des revendications 1 à 14, **caractérisée par le fait que** le milieu aqueux cosmétiquement acceptable est constitué uniquement par de l'eau ou par un mélange d'eau et d'un solvant cosmétiquement acceptable.

**16.** Composition selon la revendication 15, **caractérisée par le fait que** les le solvant est choisi les alcools inférieurs en $C_1$-$C_4$, comme l'éthanol, l'isopropanol, le tertiobutanol, le n-butanol ; les alkylèneglycols comme le propylèneglycol ou l'hexylèneglycol, le glycérol.

**17.** Composition selon l'une quelconque des revendications 1 à 16, **caractérisée par le fait qu'**elle contient en plus un ou plusieurs adjuvants choisis par les agents tensioactifs cationiques, les polymères anioniques ou non ioniques ou amphotères, les protéines, les hydrolysats de protéines, les céramides, les pseudocéramides, les huiles végétales, les acides gras, les hydroxyacides, les vitamines, les provitamines telle que le panthénol, les silicones non aminées, volatiles ou non volatiles, solubles ou insolubles dans le milieu, les filtres UV, les agents hydratants, les agents antipelliculaires ou antiséborrhéiques, les agents antichute des cheveux, les agents anti-radicaux libres, les agents opacifiants et leurs mélanges.

**18.** Utilisation d'une composition telle définie dans l'une quelconque des revendications 1 à 17 pour le nettoyage et/ou le démaquillage des matières kératiniques.

**19.** Procédé de lavage et de conditionnement des matières kératiniques telles que les cheveux consistant à appliquer sur lesdites matières mouillées une quantité efficace d'une composition telle que définie dans l'une quelconque des revendications 1 à 17, puis à effectuer un rinçage à l'eau après un éventuel temps de pause.

**Claims**

**1.** Detergent and conditioning cosmetic composition, **characterized in that** it comprises, in a cosmetically acceptable aqueous medium,

(A) at least one sulfate or sulfonate anionic surfactant, (B) at least one carboxylic anionic surfactant other than the surfactant (A), (C) at least one amphoteric surfactant and (D) at least one amino silicone,
the sulfate or sulfonate anionic surfactant/carboxylic anionic surfactant weight ratio ranging from 2 to 12,
the total amount of surfactants representing from 4% to 35% by weight relative to the total weight of the final composition.

**2.** Composition according to Claim 1, **characterized in that** the sulfate or sulfonate anionic surfactant(s) is (are) present in concentrations ranging from 1.5% to 34.4% by weight and preferably from 2% to 25% by weight relative to the total weight of the composition.

**3.** Composition according to either of Claims 1 and 2, **characterized in that** the amphoteric surfactant(s) is (are) present in concentrations ranging from 0.1% to 20% by weight and preferably from 1% to 10% by weight relative to the total weight of the composition.

**4.** Composition according to any one of Claims 1 to 3, **characterized in that** the said carboxylic anionic surfactant(s) is (are) present in concentrations ranging from 0.5% to 15% by weight and preferably from 1% to 10% by weight relative to the total weight of the composition.

**5.** Composition according to any one of Claims 1 to 4, **characterized in that** the sulfate or sulfonate anionic surfactant/amphoteric surfactant weight ratio ranges from 2 to 12, more particularly from 4 to 10 and even more preferentially from 5 to 8.

**6.** Composition according to any one of Claims 1 to 5, **characterized in that** the sulfate or sulfonate anionic surfactant/carboxylic anionic surfactant weight ratio ranges from 4 to 10.

**7.** Composition according to any one of Claims 1 to 6, **characterized in that** the carboxylic anionic surfactant/amphoteric surfactant weight ratio ranges from 0.3 to 3 and even more preferentially from 0.5 to 1.5.

**8.** Composition according to any one of Claims 1 to 7, **characterized in that** the amino silicone is chosen from:

a) polysiloxanes corresponding to formula (I):

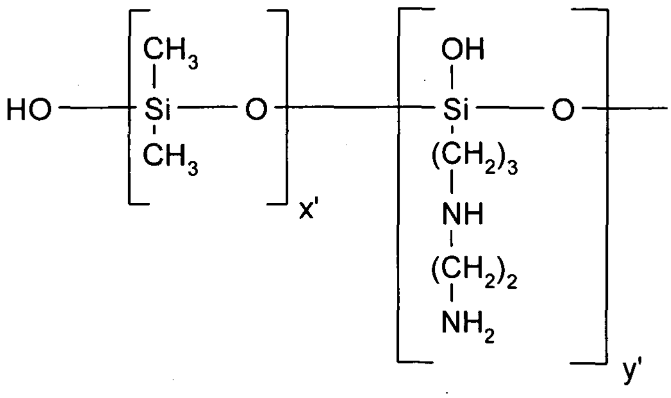

(I)

in which x' and y' are integers such that the said' weight-average molecular weight ranges from 5000 to 500 000;
b) amino silicones corresponding to the formula:

$$R'_aG_{3-a}\text{-Si }(OSiG_2)_n\text{-}(OSiG_bR'_{2-b})_m\text{-O-}SiG_{3-a}\text{-}R'_a \qquad (II)$$

in which:

G, which may be identical or different, denote a hydrogen atom or a phenyl group, OH, $C_1$-$C_8$ alkyl or $C_1$-$C_8$ alkoxy,
a, which may be identical or different, denote the number 0 or an integer from 1 to 3,
b denotes 0 or 1,
m and n are numbers such that the sum (n + m) can especially range from 1 to 2000 and in particular from 50 to 150, n possibly denoting a number from 0 to 1999 and especially from 49 to 149, and m possibly denoting a number from 1 to 2000 and especially from 1 to 10;
R', which may be identical or different, denote a monovalent radical of formula -CqH2qL in which q is a number from 2 to 8 and L is an optionally quaternized amino group chosen from the groups:

$-NR''-Q-N(R'')_2$
$-N(R'')_2$
$-N^+(R'')_3\ A^-$
$-N^+H(R'')_2\ A^-$
$-N^+H_2\ (R'')\ A^-$
$-N(R'')-Q-N^+R''H_2\ A^-$
$-NR''-Q-N^+\ (R'')_2H\ A^-$
$-NR''-Q-N^+\ (R'')_3\ A^-$,

in which R" may denote hydrogen, phenyl, benzyl, or a saturated monovalent hydrocarbon-based radical; Q denotes a linear or branched group of formula $C_rH_{2r}$, r being an integer ranging from 2 to 6 and preferably from 2 to 4; and A- represents a halide ion;

c) the amino silicones corresponding to formula (VIII):

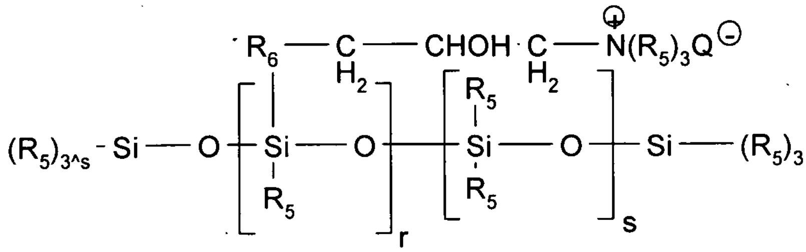

(VIII)

in which:

- $R_5$ represents a monovalent hydrocarbon-based radical containing from 1 to 18 carbon atoms;
- $R_6$ represents a divalent hydrocarbon-based radical linked to the Si via an SiC bond;
- Q- is an anion such as a halide ion or an organic acid salt;
- r represents a mean statistical value from 2 to 20;
- s represents a mean statistical value from 20 to 200;

d) the quaternary ammonium silicones of formula (IX):

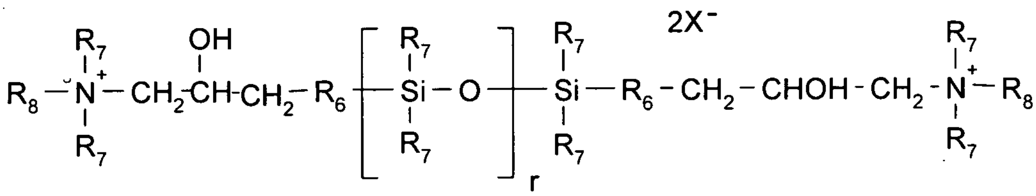

(IX)

in which:

- $R_7$, which may be identical or different, represent a monovalent hydrocarbon-based radical containing from 1 to 18 carbon atoms;
- $R_6$ represents a divalent hydrocarbon-based radical linked to the Si via an SiC bond;
- $R_8$, which may be identical or different, represent a hydrogen atom or a monovalent hydrocarbon-based radical containing from 1 to 18 carbon atoms;
- X- is an anion such as a halide ion or an organic acid salt;
- r represents a mean statistical value from 2 to 200;

e) the amino silicones of formula (X):

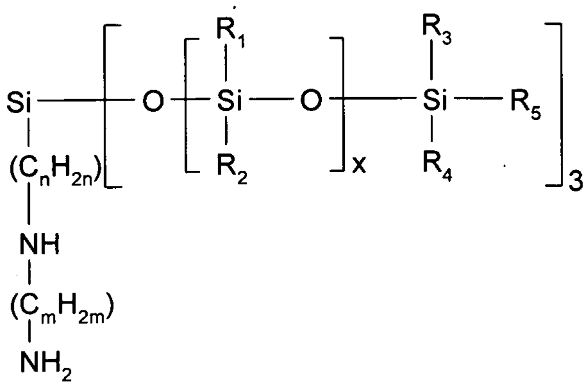

(X)

in which:

- $R_1$, $R_2$, $R_3$ and $R_4$, which may be identical or different, denote a $C_1$-$C_4$ alkyl radical or a phenyl group,
- $R_5$ denotes a $C_1$-$C_4$ alkyl radical or a hydroxyl group,
- n is an integer ranging from.1 to 5,
- m is an integer ranging from 1 to 5,

and in which x is chosen such that the amine number ranges from 0.01 to 1 meq./g;

f) the polyoxyalkylenated amino silicones of the type $(XY)_i$, X being a polysiloxane block and Y being a polyoxyalkylene block comprising at least one amine group, which may, preferably, consist of repeating units having the following general formula:

$$[SiMe_2\text{-}O\text{-}(SiMe_2O)_xSiMe_2\text{-}R\text{-}N(H)\text{-}R'\text{-}O\text{-}(C_2H_4O)_a\text{-}(C_3H_6O)_b\text{-}R'\text{-}N(H)\text{-}R\text{-}] \quad (XI)$$

in which:

- a is an integer greater than or equal to 1, preferably ranging from 5 to 200 and even more particularly from 5 to 100;
- b is an integer ranging from 0 to 200, preferably ranging from 4 to 200 and even more particularly from 5 to 100;
- x is an integer ranging from 1 to 10 000 and even more particularly from 10 to 5000;
- R, which may be identical or different, represent a divalent organic group that is linked to the adjacent silicon atom via a carbon-silica bond and to a nitrogen atom,
- R', which may be identical or different, represent a divalent organic group that is linked to the adjacent oxygen atom via a carbon-oxygen bond and to a nitrogen atom.

**9.** Composition according to the preceding claim, **characterized in that** the amino silicone is chosen from the amino silicones of formulae (I) and (II), and mixtures thereof.

**10.** Composition according to any one of the preceding claims, **characterized in that** the amino silicone is chosen from the silicones of formula (VI) and of formula (VII):

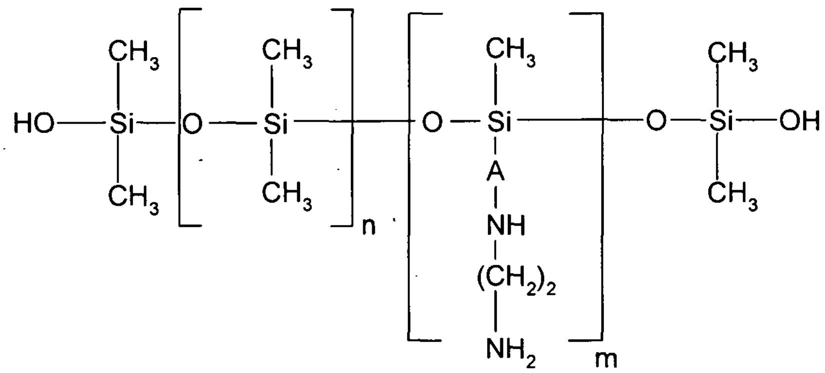

(VI)

in which:

m and n are numbers such that the sum (n + m) can range especially from 1 to 2000 and in particular from 50 to 150, n possibly denoting a number from 0 to 1999 and especially from 49 to 149, and m possibly denoting a number from 1 to 2000 and especially from 1 to 10;

A denotes a linear or branched alkylene radical containing from 4 to 8 carbon atoms and preferably 4 carbon atoms;

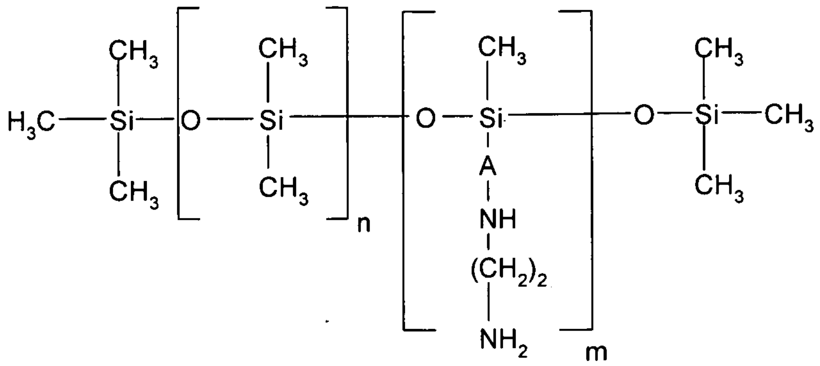

(VII)

in which:

m and n are numbers such that the sum (n + m) can range especially from 1 to 2000 and in particular from 50 to 150, n possibly denoting a number from 0 to 1999 and especially from 49 to 149, and m possibly denoting a number from 1 to 2000 and especially from 1 to 10;

A denotes a linear or branched alkylene radical containing from 4 to 8 carbon atoms and preferably 4 carbon atoms.

**11.** Composition according to any one of Claims 1 to 10, **characterized in that** the said amino silicones are present in concentrations ranging from 0.01% to 10%, preferably from 0.1% to 5% and even more particularly from 0.5% to 3% by weight relative to the total weight of the composition.

**12.** Composition according to any one of Claims 1 to 11, **characterized in that** the composition also comprises at least one cationic polymer.

**13.** Composition according to Claim 12, **characterized in that** the cationic polymer is chosen from quaternary cellulose ether derivatives, copolymers of a diallyldimethylammonium salt and of acrylamide, cationic polysaccharides and quaternary copolymers of vinylpyrrolidone and of a vinylimidazole salt.

**14.** Composition according to either of Claims 12 and 13, **characterized in that** the said cationic polymer represents from 0.005% to 10% by weight, preferably from 0.01% to 5% by weight and even more preferentially from 0.1% to 3% by weight relative to the total weight of the final composition.

**15.** Composition according to any one of Claims 1 to 14, **characterized in that** the cosmetically acceptable aqueous medium consists solely of water or of a mixture of water and of a cosmetically acceptable solvent.

**16.** Composition according to Claim 15, **characterized in that** the solvent is chosen from $C_1$-$C_4$ lower alcohols, for instance ethanol, isopropanol, tert-butanol or n-butanol; alkylene glycols, for instance propylene glycol or hexylene glycol, and glycerol.

**17.** Composition according to any one of Claims 1 to 16, **characterized in that** it also contains one or more adjuvants chosen from cationic surfactants, anionic, nonionic or amphoteric polymers, proteins, protein hydrolysates, ceramides, pseudoceramides, plant oils, fatty acids, hydroxy acids, vitamins, provitamins such as panthenol, volatile or non-volatile non-amino silicones, which are soluble or insoluble in the medium, UV-screening agents, moisturizers, antidandruff or anti-seborrhoeic agents, hair-loss counteractants, free-radical scavengers and opacifiers, and mixtures thereof.

**18.** Use of a composition as defined in any one of Claims 1 to 17, for cleansing and/or removing makeup from keratin materials.

**19.** Process for washing and conditioning keratin materials such as the hair, which consists in applying to the said wet materials an effective amount of a composition as defined in any one of Claims 1 to 17, and then in rinsing with water after an optional leave-in time.

## Patentansprüche

1. Reinigende und pflegende, kosmetische Zusammensetzung, **dadurch gekennzeichnet, dass** sie in einem kosmetisch akzeptablen wässrigen Medium enthält:

    mindestens ein anionisches grenzflächenaktives Sulfat oder Sulfonat, (B) mindestens eine anionische grenzflächenaktive Carbonsäure, die von (A) verschieden ist, (C) mindestens einen amphoteren grenzflächenaktiven Stoff und (D) mindestens ein aminiertes Silicon,

    wobei das auf das Gewicht bezogene Verhältnis des anionischen grenzflächenaktiven Sulfats oder Sulfonats und der anionischen grenzflächenaktiven Carbonsäure im Bereich von 2 bis 12 liegt, und wobei die Gesamtmenge der grenzflächenaktiven Stoffe 4 bis 35 Gew.-%, bezogen auf das Gesamtgewicht der fertigen Zusammensetzung, ausmacht.

2. Zusammensetzung nach Anspruch 1, **dadurch gekennzeichnet, dass** das oder die anionische(n) grenzflächenaktive(n) Sulfat(e) oder Sulfonat(e) in Konzentrationen von 1,5 bis 34,4 Gew.-% und vorzugsweise 2 bis 25 Gew.-%, bezogen auf das Gesamtgewicht der Zusammensetzung, enthalten ist (sind).

3. Zusammensetzung nach einem der Ansprüche 1 bis 2, **dadurch gekennzeichnet, dass** der oder die amphotere (n) grenzflächenaktive(n) Stoff(e) in Konzentrationen von 0,1 bis 20 Gew.-% und vorzugsweise 1 bis 10 Gew.-%, bezogen auf das Gesamtgewicht der Zusammensetzung, enthalten ist (sind).

4. Zusammensetzung nach einem der Ansprüche 1 bis 3, **dadurch gekennzeichnet, dass** die anionische(n) grenzflächenaktive(n) Carbonsäure(n) in Konzentrationen von 0,5 bis 15 Gew.-% und vorzugsweise 1 bis 10 Gew.-%, bezogen auf das Gesamtgewicht der Zusammensetzung, enthalten ist (sind).

5. Zusammensetzung nach einem der Ansprüche 1 bis 4, **dadurch gekennzeichnet, dass** das auf das Gewicht bezogene Verhältnis des anionischen grenzflächenaktiven Sulfats oder Sulfonats und des amphoteren grenzflächenaktiven Stoffes im Bereich von 2 bis 12, insbesondere im Bereich von 4 bis 10 und noch bevorzugter im Bereich von 5 bis 8 liegt.

6. Zusammensetzung nach einem der Ansprüche 1 bis 5, **dadurch gekennzeichnet, dass** das auf das Gewicht bezogene Verhältnis anionisches grenzflächenaktives Sulfat oder Sulfonat/anionische grenzflächenaktive Carbonsäure im Bereich von 4 bis 10 liegt.

7. Zusammensetzung nach einem der Ansprüche 1 bis 6, **dadurch gekennzeichnet, dass** das auf das Gewicht bezogene Verhältnis anionische grenzflächenaktive Carbonsäure/amphoterer grenzflächenaktiver Stoff im Bereich von 0,3 bis 3 und noch bevorzugter 0,5 bis 1,5 liegt.

8. Zusammensetzung nach einem der Ansprüche 1 bis 7, **dadurch gekennzeichnet, dass** das aminierte Silicon ausgewählt ist unter:

    a) Polysiloxanen der folgenden Formel (I):

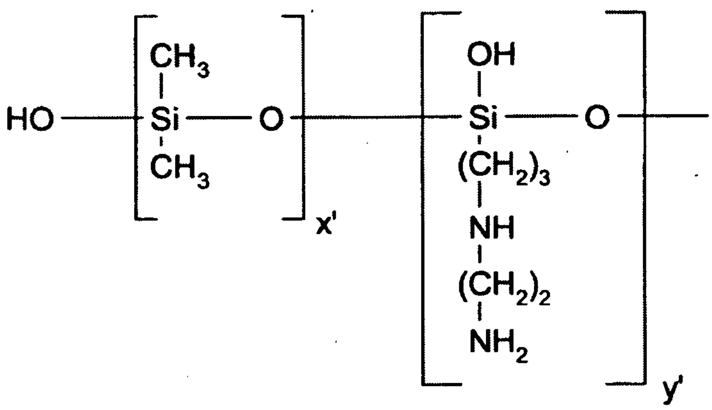

(I)

worin x' und y' ganze Zahlen sind, die so gewählt sind, dass die gewichtsmittlere Molmasse im Bereich von 5 000 bis 500 000 liegt;

b) aminierten Siliconen der Formel:

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(II)}$$

worin bedeuten:

die Gruppen G, die gleich oder verschieden sind, ein Wasserstoffatom, eine Phenylgruppe, OH, $C_{1-8}$-Alkyl oder $C_{1-8}$-Alkoxy,
die Indices a, die gleich oder verschieden sind, 0 oder eine ganze Zahl von 1 bis 3,
b 0 oder 1,
m und n Zahlen, die so sind, dass die Summe (n + m) insbesondere im Bereich von 1 bis 2 000 und besonders 50 bis 150 liegen kann, wobei n eine Zahl von 0 bis 1 999 und
insbesondere 49 bis 149 bedeuten kann und m eine Zahl von 1 bis 2 000 und insbesondere 1 bis 10 sein kann;
die Gruppen R', die gleich oder verschieden sind, eine einwertige Gruppe der Formel $-CqH_2qL$, worin q eine ganze Zahl von 2 bis 8 ist und L eine gegebenenfalls quaternisierte aminierte Gruppe ist, die unter den folgenden Gruppen ausgewählt ist:

**$-NR''-Q-N(R'')_2$**
**$-N(R'')_2$**
**$-N^+(R'')_3A-$**
**$-N^+H(R'')_2\ A-$**
**$-N^+H_2(R'')\ A-$**
**$-N(R'')-Q-N^+R''H_2\ A-$**
**$-NR''-Q-N^+\ (R'')_2H\ A-$**
**$-NR''-Q-N^+\ (R'')_3\ A-$,**

worin die Gruppe R" Wasserstoff, Phenyl, Benzyl oder eine gesättigte einwertige Kohlenwasserstoffgruppe bedeuten kann; Q eine geradkettige oder verzweigte Gruppe der Formel $C_rH_{2r}$ ist, wobei r eine ganze Zahl von 2 bis 6 und vorzugsweise 2 bis 4 bedeutet; und A- ein Halogenid bedeutet,

c) aminierten Siliconen der folgenden Formel (VIII):

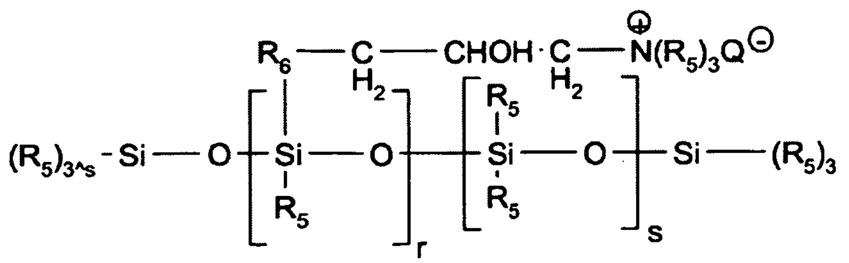

**(VIII)**

wobei in der Formel:

$R_5$ bedeutet eine einwertige Kohlenwasserstoffgruppe mit 1 bis 18 Kohlenstoffatomen;
$R_6$ bedeutet eine zweiwertige Kohlenwasserstoffgruppe, die über eine Si-C-Bindung an Si gebunden ist;
Q- ist ein Anion, beispielsweise ein Halogenid oder das Salz einer organischen Säure;
r bedeutet einen statistischen Mittelwert von 2 bis 20; s bedeutet einen statistischen Mittelwert von 20 bis 200;

d) quartären Ammoniumsiliconen der folgenden Formel (IX):

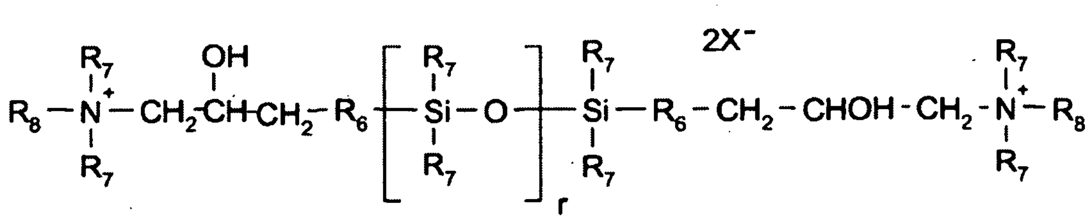

(IX)

wobei in der Formel:

die Gruppen $R_7$, die gleich oder verschieden sind, bedeuten eine einwertige Kohlenwasserstoffgruppe mit 1 bis 18 Kohlenstoffatomen;
$R_6$ bedeutet eine zweiwertige Kohlenwasserstoffgruppe, die über eine Si-C-Bindung an Si gebunden ist;
die Gruppen $R_8$, die gleich oder verschieden sind, bedeuten ein Wasserstoffatom, eine einwertige Kohlenwasserstoffgruppe mit 1 bis 18 Kohlenstoffatomen;
X- ist ein Anion, beispielsweise ein Halogenid oder ein organisches Säuresalz;
r bedeutet einen statistischen Mittelwert von 2 bis 200;

e) aminierten Siliconen der Formel (X):

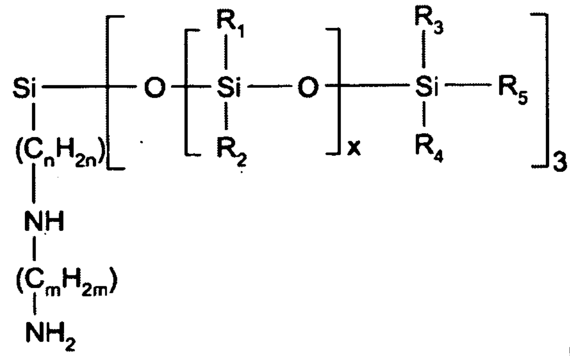

(X)

wobei in der Formel:

- $R_1$, $R_2$, $R_3$ und $R_4$, die gleich oder verschieden sind, bedeuten eine $C_{1-4}$-Alkylgruppe oder eine Phenylgruppe,
- $R_5$ bedeutet eine $C_{1-4}$-Alkylgruppe oder eine Hydroxygruppe,
- n ist eine ganze Zahl von 1 bis 5,
- m ist eine ganze Zahl von 1 bis 5,

wobei x so gewählt ist, dass der Amin-Index im Bereich von 0,01 1 bis 1 meq/g liegt;
f) polyalkoxylierten aminierten Siliconen vom Typ $(XY)_i$, wobei X ein Polysiloxanblock ist und Y ein Polyoxyalkylenblock ist, der mindestens eine Aminogruppe aufweist, die vorzugsweise aus Wiederholungseinheiten der folgenden allgemeinen Formel bestehen können:

$$[SiMe_2\text{-}O\text{-}(SiMe_2O)_xSiMe_2 - R\text{-}N(H)\text{-}R'\text{-}O - (C_2H_4O)e - (C_3H_6O)_b - R'\text{-}N(H)\text{-}R\text{-}]$$ **(XI)**

in der Formel:

- a ist eine ganze Zahl größer oder gleich 1, vorzugsweise im Bereich von 5 bis 200 und besonders bevorzugt im Bereich von 5 bis 100,

- b ist eine ganze Zahl im Bereich von 0 bis 200, vorzugsweise im Bereich von 4 bis 200 und besonders bevorzugt im Bereich von 5 bis 100,
- x ist eine ganze Zahl im Bereich von 1 bis 10 000 und besonders bevorzugt 10 bis 5 000,
- die Gruppen R, die gleich oder verschieden sind, bedeuten eine zweiwertige organische Gruppe, die über eine Kohlenstoff-Silicium-Bindung an das angrenzende Siliciumatom und an ein Stickstoffatom gebunden ist,
- die Gruppen R', die gleich oder verschieden sind, bedeuten eine zweiwertige organische Gruppe, die über eine Kohlenstoff-Sauerstoff-Bindung an das angrenzende Sauerstoffatom und an ein Stickstoffatom gebunden ist.

**9.** Zusammensetzung nach dem vorhergehenden Anspruch, **dadurch gekennzeichnet, dass** das aminierte Silicon unter den aminierten Siliconen der Formel (I) und (II) und deren Gemischen ausgewählt ist.

**10.** Zusammensetzung nach einem der vorhergehenden Ansprüche, **dadurch gekennzeichnet, dass** das aminierte Silicon unter den Siliconen der Formel (VI) und der Formel (VII) ausgewählt ist:

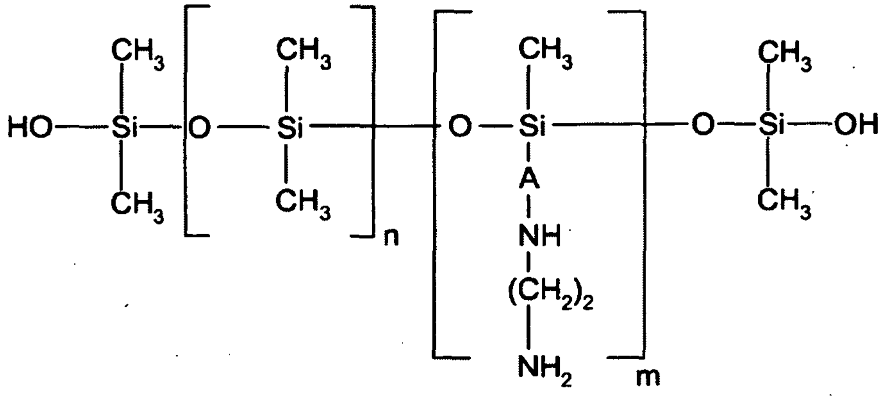

(VI)

in der Formel:

m und n bedeuten Zahlen, die so sind, dass die Summe (n + m) insbesondere im Bereich von 1 bis 2 000 und besonders 50 bis 150 liegen kann, wobei n eine Zahl von 0 bis 1 999 und insbesondere 49 bis 149 bedeuten kann und m eine Zahl von 1 bis 2 000 und insbesondere 1 bis 10 sein kann;
A bedeutet eine geradkettige oder verzweigte Alkylengruppe mit 4 bis 8 Kohlenstoffatomen und vorzugsweise 4 Kohlenstoffatomen;

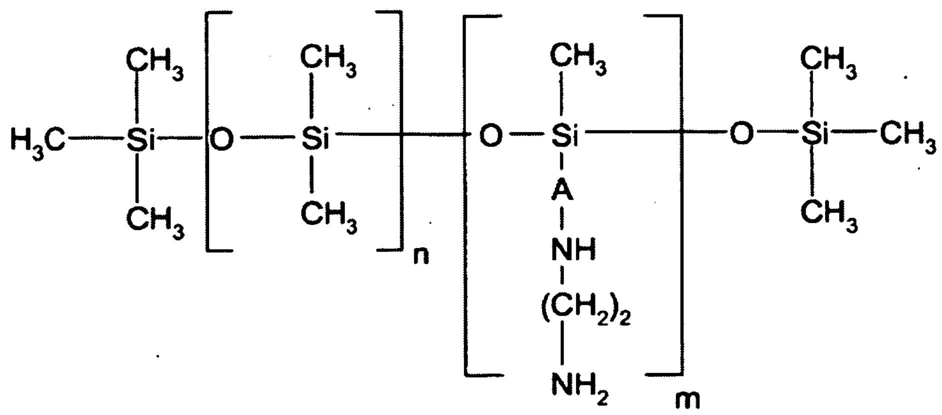

(VII)

in der Formel:

m und n bedeuten Zahlen, die so sind, dass die Summe (n + m) insbesondere im Bereich von 1 bis 2 000 und besonders 50 bis 150 liegen kann, wobei n eine Zahl von 0 bis 1 999 und insbesondere 49 bis 149 bedeuten kann und m eine Zahl von 1 bis 2 000 und insbesondere 1 bis 10 sein kann;

A bedeutet eine geradkettige oder verzweigte Alkylengruppe mit 4 bis 8 Kohlenstoffatomen und vorzugsweise 4 Kohlenstoffatomen.

**11.** Zusammensetzung nach einem der Ansprüche 1 bis 10, **dadurch gekennzeichnet, dass** die aminierten Silicone in Konzentrationen von 0,01 1 bis 10 %, vorzugsweise 0,1 bis 5 % und noch bevorzugter 0,5 bis 3 Gew.-%, bezogen auf das Gesamtgewicht der Zusammensetzung, enthalten sind.

**12.** Zusammensetzung nach einem der Ansprüche 1 bis 11, **dadurch gekennzeichnet, dass** sie ferner mindestens ein kationisches Polymer enthält.

**13.** Zusammensetzung nach Anspruch 12, **dadurch gekennzeichnet, dass** das kationische Polymer unter den quartären Celluloseetherderivaten, Copolymeren von Diallyldimethylammoniumsalzen und Acrylamid, kationischen Polysacchariden, quartären Copolymeren von Vinylpyrrolidon und einem Vinylimidazolsalz ausgewählt ist.

**14.** Zusammensetzung nach einem der Ansprüche 12 und 13, **dadurch gekennzeichnet, dass** das kationische Polymer 0,005 bis 10 Gew.-%, vorzugsweise 0,01 bis 5 Gew.-% und noch bevorzugter 0,1 bis 3 Gew.-% des Gesamtgewichts der fertigen Zusammensetzung ausmacht.

**15.** Zusammensetzung nach einem der Ansprüche 1 bis 14, **dadurch gekennzeichnet, dass** das kosmetisch akzeptable wässrige Medium aus Wasser oder aus einem Gemisch von Wasser und einem kosmetisch akzeptablen Lösungsmittel besteht.

**16.** Zusammensetzung nach Anspruch 15, **dadurch gekennzeichnet, dass** das Lösungsmittel unter den niederen Alkoholen mit 1 bis 4 Kohlenstoffatomen, wie Ethanol, Isopropanol, tert-Butanol, n-Butanol; Alkylenglycolen, wie Propylenglycol oder Hexylenglycol, Glycerol ausgewählt ist.

**17.** Zusammensetzung nach einem der Ansprüche 1 bis 16, **dadurch gekennzeichnet, dass** sie ferner einen oder mehrere Zusatzstoffe enthält, die unter den kationischen grenzflächenaktiven Stoffen, anionischen, nichtionischen oder amphoteren Polymeren, Proteinen, Proteinhydrolysaten, Ceramiden, Pseudoceramiden, pflanzlichen Ölen, Fettsäuren, Hydroxysäuren, Vitaminen, Provitaminen wie Panthenol, nicht aminierten Siliconen, die flüchtig oder nicht flüchtig und in dem Medium löslich oder unlöslich sind, UV-Filtern, Hydratisierungsmitteln, Antischuppenmitteln, Wirkstoffen gegen Seborrhoe, Wirkstoffen gegen Haarausfall, Radikalfängern für freie Radikale, Trübungsmitteln und deren Gemischen ausgewählt sind.

**18.** Verwendung einer Zusammensetzung nach einem der Ansprüche 1 bis 17 für die Reinigung und/oder zum Abschminken von Keratinsubstanzen.

**19.** Verfahren für die Reinigung und Pflege von Keratinsubstanzen, wie Haaren, das darin besteht, eine wirksame Menge einer Zusammensetzung nach einem der Ansprüche 1 bis 17 auf die feuchten Keratinsubstanzen aufzutragen und anschließend nach einer gegebenenfalls abgewarteten Einwirkzeit mit Wasser zu spülern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2003134760 A
- EP 0739625 A
- WO 974622 A
- US 2003147842 A
- US 6153569 A
- US 22003157049 A
- US 2528378 A
- US 2781354 A
- EP 95238 A
- US 4185087 A
- EP 0530974 A
- EP 0337354 A
- FR 2270846 A
- FR 2383660 A
- FR 2598611 A
- FR 2470596 A
- FR 2519863 A